# EUROPÄISCHE PATENTANMELDUNG

(11) **EP 1 787 603 A1**
(43) Veröffentlichungstag der Anmeldung: **23.05.2007**
(21) Anmeldenummer: 05025291.5
(22) Anmeldetag: 18.11.2005
(51) Int. Cl.: A61F 2/40

(54) **Basisplattform für ein künstliches Gelenk**

(71) Anmelder: Zimmer GmbH, 8404 Winterthur (CH)
(72) Erfinder: Baum, Ines, 78467 Konstanz (DE); Rauscher, Markus, 8266 Steckborn (CH); Wendt, Peter, 8542 Wiesendangen (CH)
(74) Vertreter: Manitz, Finsterwald & Partner GbR

(57) **Zusammenfassung**

Die Erfindung betrifft eine Basisplattform (11) für ein künstliches Gelenk, insbesondere für ein künstliches Schultergelenk, wobei die Plattform am Knochen verankerbar ist und als Träger für eine künstliche Gelenkkomponente dient, die sowohl in Form einer Schalenkomponente (15) für eine anatomische Konfiguration als auch in Form einer Kugelkomponente (17) für eine inverse Konfiguration vorgesehen sein kann, und wobei an der Plattform verschiedene Gelenkkomponenten montierbar sind.

## Beschreibung

Die Erfindung betrifft eine Basisplattform für ein künstliches Gelenk. Ferner betrifft die Erfindung eine entweder als Schale oder Pfanne oder als Kugel oder Kopf ausgebildete künstliche Gelenkkomponente. Des Weiteren betrifft die Erfindung ein Gelenkteil mit einer derartigen Basisplattform und mit einer derartigen Gelenkkomponente. Ferner betrifft die Erfindung einen Bausatz zur Bildung unterschiedlicher Gelenkteile mit zumindest einer derartigen Plattform und mit mehreren unterschiedlichen derartigen Gelenkkomponenten. Die Erfindung betrifft außerdem ein Verfahren zum Implantieren eines künstliches Gelenks, das ein erfindungsgemäßes Gelenkteil mit einer erfindungsgemäßen Plattform umfasst.

Die Erfindung ist grundsätzlich für alle Gelenke anwendbar und wird nachstehend am Beispiel eines Schultergelenkes beschrieben, das ein mögliches Anwendungsfeld für die Erfindung darstellt.

Bei Schultergelenken, aber auch bei anderen Gelenken, besteht ein Problem darin, dass die Auswahl des passenden Implantats und der Art und Weise der Implantation entscheidend davon abhängen, in welchem Zustand sich die beteiligten Knochen befinden, wobei auch der Zustand der Muskeln, insbesondere der Rotatorenmanschette, eine Rolle spielt. Beim Schultergelenk kommt es insbesondere auf den Zustand des Schulterblatts und dabei vor allem der Gelenkpfanne (Glenoid) an, die beim gesunden Gelenk mit dem Kopf des Oberarms (Humerus) zusammenwirkt. Die Notwendigkeit für einen teilweisen oder vollständigen Ersatz des Schultergelenks kann aus unterschiedlichen Gründen bestehen. Typische Ursachen sind beispielsweise fortgeschrittener Verschleiß der Gelenkflächen oder Frakturen z.B. aufgrund eines Unfalls.

Je nach Art und Ausmaß der Schädigung kann auch eine so genannte inverse Prothesenkonfiguration angezeigt sein, bei welcher die künstliche Gelenkkugel und die künstliche Gelenkschale bezüglich ihrer Positionen in einem natürlichen Gelenk vertauscht sind.

Problematisch ist, dass der Operateur häufig erst während der Operation die richtige Entscheidung über die Art der Prothese und deren Anbringung treffen kann. Ferner kann sich eine zunächst getroffene Auswahl zwar für eine Erstversorgung als richtig erweisen, sich später aber als änderungsbedürftig herausstellen, was eine erneute Operation und die damit verbundenen Risiken und Belastungen des Patienten zur Folge hat.

Es besteht daher Bedarf an Möglichkeiten, die Implantation von Gelenkprothesen so einfach und flexibel wie möglich sowie mit möglichst geringen Risiken und Belastungen für den Patienten durchzuführen. Hier setzt die Erfindung an.

Ein Aspekt der Erfindung betrifft eine Basisplattform mit den Merkmalen des Anspruchs 1.

Die erfindungsgemäße Plattform kann als Träger sowohl für eine künstliche Schale oder Pfanne (anatomische Konfiguration) als auch für eine Kugel oder einen Kopf (inverse Konfiguration) dienen und wird somit am Knochen verankert, der insbesondere die natürliche Gelenkpfanne bildet, im Fall eines Schultergelenks also am Schulterblatt, d.h. insbesondere an dessen Glenoid.

Erfindungsgemäß sind an der Plattform verschiedene Gelenkkomponenten montierbar. Unter "montierbar" ist dabei zu verstehen, dass eine Gelenkkomponente bei am Knochen verankerter Plattform - also während einer Operation - an der Plattform angebracht werden kann, wobei es auch möglich ist, bei am Knochen verankerter Plattform eine an der Plattform montierte Gelenkkomponente gegen eine andere Gelenkkomponente auszutauschen. Erfindungsgemäß kann unter Beibehaltung der Basisplattform eine künstliche Schalenkomponente gegen eine künstliche Kugelkomponente ausgetauscht werden, und umgekehrt, da die Plattform sowohl für eine anatomische als auch für eine inverse Konfiguration ausgelegt ist.

Ein weiterer Aspekt der Erfindung betrifft eine Gelenkkomponente für ein künstliches Gelenk, insbesondere für ein künstliches Schultergelenk, mit den Merkmalen des Anspruchs 17.

Ein weiterer Aspekt der Erfindung betrifft ein Gelenkteil für ein künstliches Gelenk, wobei das Gelenkteil eine erfindungsgemäße Plattform sowie eine an der Plattform verankerte erfindungsgemäße Gelenkkomponente umfasst. Das erfindungsgemäße Gelenkteil kann somit sowohl in einer anatomischen Konfiguration als auch in einer inversen Konfiguration realisiert werden.

Gemäß einem weiteren Aspekt betrifft die Erfindung einen Bausatz zur Bildung unterschiedlicher Gelenkteile, wobei der Bausatz wenigstens eine erfindungsgemäße Plattform sowie mehrere unterschiedliche künstliche Gelenkkomponenten umfasst, die jeweils als Schalenkomponente oder als Kugelkomponente ausgebildet sind. Dieser Bausatz ermöglicht es dem Operateur, während der Operation das jeweils am besten passende Gelenkteil zu bilden. Dies gilt sowohl für eine Erstversorgung als auch für eine gegebenenfalls erforderliche Revision.

Ein weiterer Aspekt der Erfindung betrifft ein Verfahren zum Implantieren eines künstlichen Gelenks, bei dem ein erfindungsgemäßes Gelenkteil in einer Ausgangskonfiguration vorliegt und diese Ausgangskonfiguration in eine Endkonfiguration geändert wird, indem eine an der erfindungsgemäßen Plattform des Gelenkteils verankerte künstliche Gelenkkomponente gegen eine andere künstliche Gelenkkomponente ausgetauscht wird.

Dabei ist es insbesondere möglich, dass eine anatomische Konfiguration in eine inverse Konfiguration geändert wird, und umgekehrt. Wenn die Konfigurationsänderung im Rahmen einer Revision erfolgen soll, ist erfindungsgemäß von Vorteil, dass die Plattform am Knochen verankert bleiben kann.

Weiterbildungen der Erfindung sind auch in den abhängigen Ansprüchen, der Beschreibung sowie der Zeichnung angegeben.

Die Plattform kann einen Zapfen aufweisen, durch den eine Befestigung der Plattform am Knochen erfolgen kann (Primärverankerung) und der einfach in eine entsprechend vorbereitete Vertiefung im Knochen eingeschlagen wird. Der Zapfen kann sich, insbesondere konusförmig, verjüngen. Die Primärverankerung kann gegebenenfalls durch zusätzliche Verankerungsschrauben unterstützt werden (Sekundärverankerung). Bei diesen Verankerungsschrauben kann es sich um polyaxiale Schrauben handeln, für welche die erfindungsgemäße Plattform mit entsprechenden Schraubenaufnahmen zusätzlich zu einem Zapfen versehen sein kann. Derartige polyaxiale Schraubverankerungen sind in Verbindung mit Knochenplatten zur Osteosynthese grundsätzlich bekannt. Die erfindungsgemäße Basisplattform kann mittels der polyaxialen Verankerungsschrauben in einer hinsichtlich der jeweiligen Gegebenheiten optimierten Orientierung der Verankerungsschrauben am Knochen verankert werden.

Hierdurch wird ein hohes Maß an Flexibilität bei Gelenkoperationen geschaffen, da die Plattform als Basis sowohl in einer anatomischen Konfiguration als auch in einer inversen Konfiguration dienen kann, und da aufgrund der Möglichkeit, zusätzlich zu einem (Konus-)Zapfen Schraubenaufnahmen für polyaxiale Verankerungsschrauben vorzusehen, auch bei schwierigen Verhältnissen eine sichere und zuverlässige Verankerung am Knochen möglich ist.

Der Zapfen kann zumindest über einen Teil seiner Länge eine von einer Kreisform abweichende äußere Querschnittsform aufweisen. Die Querschnittsform kann beispielsweise oval oder elliptisch sein.

Eine Mittelachse des Zapfens und Mittelachsen wenigstens zweier Schraubenaufnahmen können in einer gemeinsamen Hauptebene liegen. Die Plattform kann eine von einer Kreisform abweichende Grundform mit einer Symmetrieebene aufweisen, die mit der gemeinsamen Hauptebene zusammenfällt.

Wenn der Zapfen zumindest über einen Teil seiner Länge eine ovale oder elliptische äußere Querschnittsform aufweist, deren eine, insbesondere lange, Hauptachse in einer gemeinsamen Hauptebene liegt, und wenn die Plattform eine von einer Kreisform abweichende Grundform mit einer Symmetrieebene aufweist, die mit der gemeinsamen Hauptebene zusammenfällt, dann ergibt sich insgesamt eine Basisplattform mit einer wohldefinierten und eindeutig identifizierbaren Vorzugsrichtung. Dies ermöglicht es dem Operateur nicht nur, die Plattform relativ zum Knochen, sondern vor allem auch die an der Plattform anzubringende künstliche Gelenkkomponente relativ zur Plattform eindeutig auszurichten. Dies ist insbesondere dann von Vorteil, wenn die Gelenkkomponente mit einer Asymmetrie oder Exzentrizität versehen ist, die es für den Operateur erforderlich macht, die Gelenkkomponente in einer bestimmten Lage relativ zum Knochen zu fixieren.

Wenigstens zwei Schraubenaufnahmen der Plattform können derart ausgeführt sein, dass ihre Mittelachsen in unterschiedliche Richtungen weisen. Ein Verspreizen oder Verspannen der Plattform am Knochen mittels von den Schraubenaufnahmen gehaltener polyaxialer Verankerungsschrauben wird hierdurch erleichtert.

Wenn die Schraubenaufnahmen jeweils einen Verankerungskragen aufweisen, der auf der gleichen Seite wie der Zapfen von der Plattform absteht, dann kann hierdurch die mit dem konusförmigen Zapfen hergestellte Primärverankerung unterstützt werden. Wenn die Mittelachsen der Schraubenaufnahmen und damit der Verankerungskragen in unterschiedliche Richtungen weisen, dann kann bereits durch die Verankerungskragen eine Verspreizung bzw. Verspannung erreicht werden, auch wenn keine polyaxialen Verankerungsschrauben verwendet werden.

Die Schraubenaufnahmen können Mittel aufweisen, mit denen die polyaxialen Verankerungsschrauben innerhalb vorgegebener Grenzen in einer beliebigen Orientierung relativ zur Plattform fixierbar sind. Die Mittel können jeweils einen Gewindeabschnitt für eine Fixiermutter umfassen, die mit einem Kopf der polyaxialen Verankerungsschraube zusammenwirkt.

Die Plattform kann aus Metall hergestellt sein.

Die Plattform kann einstückig ausgeführt sein.

Die Plattform kann im Randbereich ihrer bei verankerter künstlicher Gelenkkomponente der Gelenkkomponente zugewandten Seite wenigstens eine von außen zugängliche, insbesondere taschenartige Ablöseaussparung aufweisen, in die oder an der ein Ablöseinstrument zum Lösen der Verankerung der Gelenkkomponente an der Plattform einbringbar bzw. ansetzbar ist. Analog kann - alternativ oder zusätzlich - eine solche Ablöseaussparung auch an der künstlichen Gelenkkomponente ausgebildet sein.

Der Zapfen der erfindungsgemäßen Basisplattform kann hohl ausgeführt und zur Aufnahme eines Fixierabschnitts der künstlichen Gelenkkomponente ausgebildet sein. Die Gelenkkomponente ist dann über ihren Fixierabschnitt an der Plattform verankerbar. Der Fixierabschnitt der Gelenkkomponente kann eine sich, insbesondere konusförmig, verjüngende Au-ßenseite aufweisen.

Die innere Querschnittsformgebung des Zapfens kann der äußeren Querschnittsformgebung des Zapfens entsprechen.

Wenn der Zapfen der Plattform hohl ausgeführt ist und zur Aufnahme eines Fixierabschnitts der künstlichen Gelenkkomponente dient, um die künstliche Gelenkkomponente über ihren Fixierabschnitt an der Plattform zu verankern, dann kann der Zapfen zumindest über einen Teil seiner Länge eine von einer Kreisform abweichende innere Querschnittsform auf weisen. Diese innere Querschnittsform kann oval oder elliptisch sein.

Bei der Verbindung zwischen Zapfen der Plattform und Fixierabschnitt der künstlichen Gelenkkomponente kann es sich um eine Klemm- oder Presssitzverbindung, insbesondere um einen Kegelpresssitz, handeln, wobei alternativ auch eine Rast- oder Schnappverbindung vorgesehen sein kann. Allgemein kann die Verbindung zwischen Zapfen und Fixierabschnitt sowohl kraftschlüssig als auch formschlüssig ausgebildet sein. Die Verbindung kann selbst hemmend ausgebildet sein.

Der Vorteil einer kraftschlüssigen Verbindung, insbesondere eines Presssitzes, zwischen Zapfen und Fixierabschnitt besteht darin, dass auf einfache Weise eine besonders feste und stabile Verbindung erzielt werden kann, bei der insbesondere die Gefahr eines Partikelabriebs und/oder einer frühzeitigen Lockerung verringert ist. Bei entsprechender geometrischer Ausgestaltung des Zapfens und des Fixierabschnitts kann die Verbindung zwischen Plattform und Gelenkkomponente besonders einfach hergestellt werden, indem beispielsweise durch einen einzigen Schlag mit definierter Schlagkraft auf die Gelenkkomponente deren Fixierabschnitt in den Zapfen der Plattform eingeschlagen wird. Im Fall einer Verbindung mit sich verjüngenden Teilen, insbesondere einer konischen Verbindung, kann hierdurch eine gleichmäßige Verspannung entlang der gesamten wirksamen Innenseite des Zapfens erzielt werden.

Der Zapfen und der Fixierabschnitt können derart ausgebildet sein, dass die Innenseite des Zapfens und die Außenseite des Fixierabschnitts einander entweder über ihren gesamten Umfang oder lediglich an mehreren diskret über den Umfang verteilten, linien- oder streifenförmigen Bereichen berühren. Diese Berührung braucht nicht über die gesamte Länge des Zapfens bzw. Fixierabschnitts zu erfolgen, wobei dies aber auch nicht ausgeschlossen ist.

Die künstliche Gelenkkomponente kann einen Schalen- oder Kugelabschnitt sowie einen Fixierabschnitt aufweisen. Der Fixierabschnitt kann sowohl mittig als auch exzentrisch bezüglich des Schalen- bzw. Kugelabschnitts angeordnet ist. Die Art der Anordnung kann von den Abmessungen des Schalen- bzw. Kugelabschnitts abhängig sein, z.B. vom Durchmesser der Kugel. Bei einer exzentrischen Anordnung kommt es beim Einsetzen der künstlichen Gelenkkomponente auf deren Orientierung relativ zum Knochen und damit - wenn die Gelenkkomponente nicht in beliebiger Orientierung relativ zur Plattform an dieser verankert werden kann - auf die Orientierung der Plattform relativ zum Knochen an.

Die künstliche Gelenkkomponente kann mehrteilig ausgebildet sein.

Dabei kann die Gelenkkomponente ein separates Fixierstück umfassen, das einen Fixierabschnitt zur Verankerung an der Plattform bildet und mit einem Schalen- oder Kugelabschnitt der Gelenkkomponente fest verbunden ist. Durch diese Mehrteiligkeit der künstlichen Gelenkkomponente ist es insbesondere möglich, den Schalen- oder Kugelabschnitt einerseits und das Fixierstück andererseits aus unterschiedlichen Materialien herzustellen. Der Schalen- oder Kugelabschnitt kann somit gezielt auf sein jeweiliges Gegenstück, mit dem es die eigentliche Gelenkverbindung bildet, abgestimmt werden, wohingegen das Fixierstück hinsichtlich der Verankerung des Schalen- oder Kugelabschnitts an der Plattform optimiert sein kann.

Insbesondere kann das separate Fixierstück im Hinblick auf eine optimale Materialpaarung mit dem Zapfen der Plattform ausgeführt werden. Dabei kann der Schalen- oder Kugelabschnitt aus Kunststoff und das separate Fixierstück aus Metall hergestellt sein. Wenn die erfindungsgemäße Plattform aus Metal hergestellt ist, kann auf diese Weise eine Metall/Metall-Verbindung zwischen dem separaten Fixierstück der Gelenkkomponente einerseits und dem Zapfen der Plattform andererseits realisiert werden.

Eine solche Materialpaarung ist für die Herstellung einer kraftschlüssigen Verbindung, insbesondere einer Presssitzverbindung, von Vorteil.

Das separate Fixierstück kann in Bezug auf Kräfte, die bei der Herstellung einer kraftschlüssigen Verbindung mit den Zapfen der Plattform wirksam sind, nachgiebig ausgebildet sein. Eine solche Nachgiebigkeit kann dadurch realisiert werden, dass das separate Fixierstück mit einem oder mehreren Längsschlitzen versehen wird.

Das Fixierstück kann einen flanschartigen Fußabschnitt aufweisen, über den das Fixierstück mit dem Schalen- oder Kugelabschnitt der Gelenkkomponente verbunden ist.

Die Verbindung zwischen dem Fixierstück und dem Schalen- oder Kugelabschnitt der Gelenkkomponente kann ohne zusätzliche Befestigungsmittel erfolgen. Der Schalen- oder Kugelabschnitt kann aus Kunststoff hergestellt und mit dem Fixierstück, insbesondere mit dessen flanschartigem Fußabschnitt, durch Verpressen verbunden sein. Hierzu kann der Fußabschnitt mit einer oder mehreren Bohrungen versehen sein, mit denen das Kunststoffmaterial der Gelenkkomponente durch das Verpressen eine innige formschlüssige Verbindung eingehen kann. Das Kunststoffmaterial der Gelenkkomponente kann für das Verpressen durch Erwärmen aufgeweicht werden.

Der Schalen- oder Kugelabschnitt kann auch aus Metall hergestellt sein.

Die Plattform und die Gelenkkomponente können derart ausgebildet sein, dass die Verankerung ohne Beschädigung der Plattform gelöst werden kann.

Hierzu kann die Gelenkkomponente mit wenigstens einem Gewindeabschnitt für ein einschraubbares Abdrückinstrument versehen sein. Der Gewindeabschnitt kann in Form eines Innengewindes des Fixierabschnitts und/oder einer Gewindebohrung in einem flanschartigen Fußabschnitt eines separaten Fixierstücks vorgesehen sein.

Alternativ oder zusätzlich können die Gelenkkomponente und die Plattform gemeinsam wenigstens eine von außen zugängliche Ablöseaussparung begrenzen, in die oder an der ein Ablöseinstrument zum Lösen der Verbindung zwischen Gelenkkomponente und Plattform einbringbar bzw. ansetzbar ist. Die Aussparung kann entweder an der Gelenkkomponente oder an der Plattform ausgebildet sein, wobei es auch möglich ist, dass an der Gelenkkomponente und der Plattform ausgebildete Teilaussparungen im zusammengesetzten Zustand gemeinsam eine Ablöseaussparung bilden.

Die künstliche Gelenkkomponente kann mit Verankerungsvorsprüngen versehen sein, die in die Schraubenaufnahmen der Plattform eingreifen. Eine solche Gelenkkomponente kann verwendet werden, wenn eine Primärverankerung der Plattform am Knochen über ihren Zapfen ausreicht und die Schraubenaufnahmen nicht für Verankerungsschrauben benötigt werden.

Die Erfindung wird im Folgenden beispielhaft unter Bezugnahme auf die Zeichnung beschrieben. Es zeigen:
- Fig. 1 und 2: ein künstliches Schultergelenk in einer anatomischen (Fig. 1) bzw. inversen (Fig. 2) Konfiguration,
- Fig. 3: verschiedene Ansichten eines erfindungsgemäßen Gelenkteils für eine inverse Konfiguration gemäß einem Ausführungsbeispiel der Erfindung, und
- Fig. 4 bis 8: jeweils verschiedene Ansichten eines weiteren Ausführungsbeispiels eines erfindungsgemäßen Gelenkteils für eine anatomische Konfiguration.

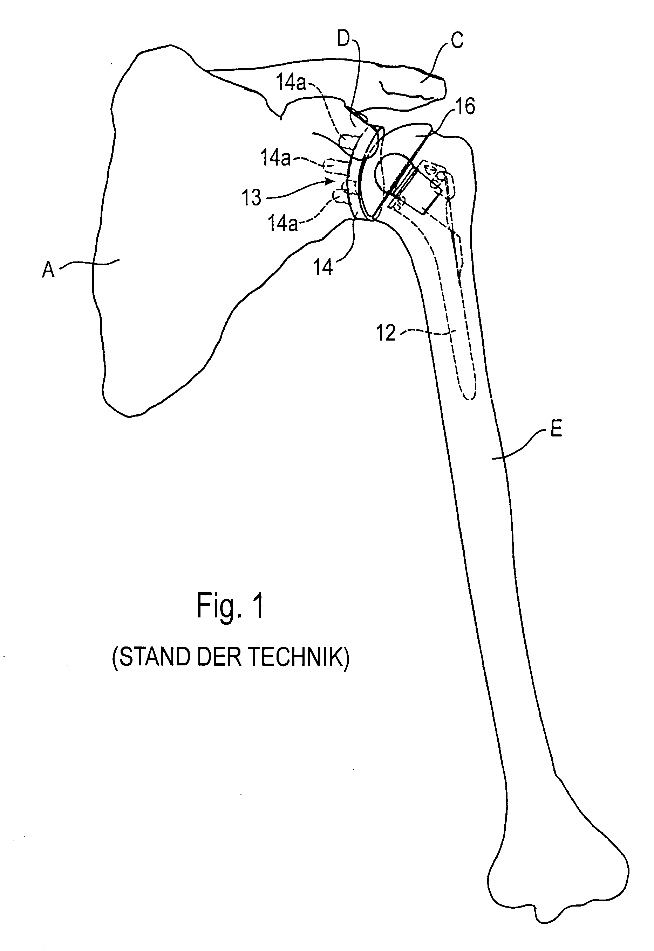

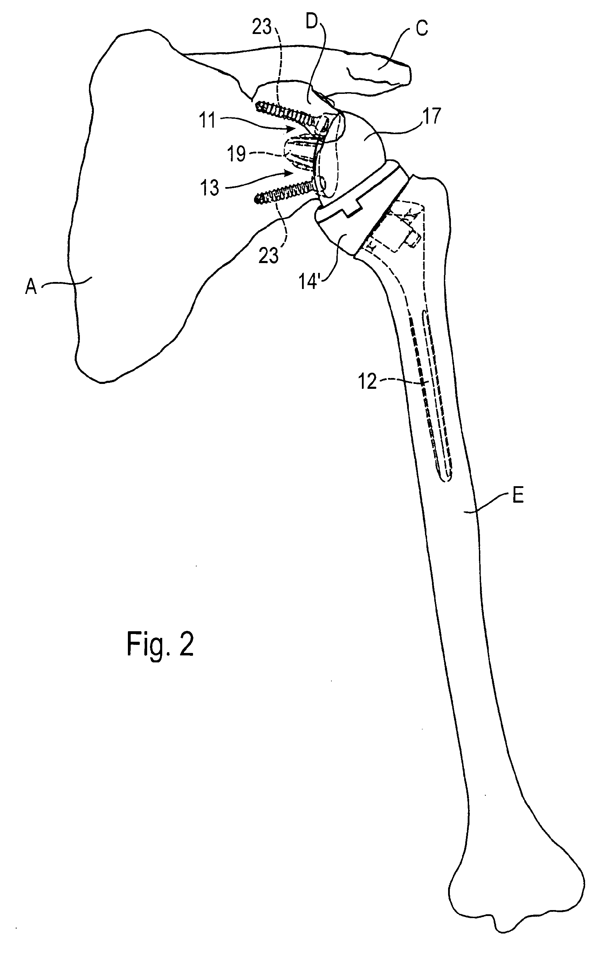

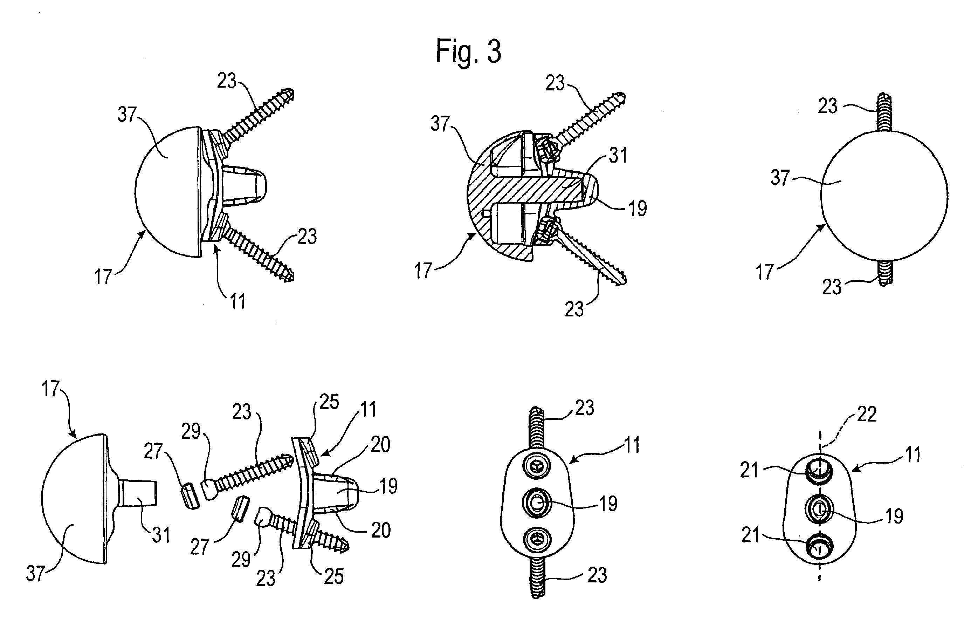

Wie eingangs bereits erwähnt, ist die Erfindung grundsätzlich in Verbindung mit beliebigen Gelenken einsetzbar. Ein mögliches Einsatzgebiet ist das Schultergelenk. Bevor anhand der Fig. 3 bis 8 verschiedene Ausführungsformen der Erfindung im Detail vorgestellt werden, soll zunächst anhand der Fig. 1 und 2 ein einleitender Überblick gegeben werden, und zwar am Beispiel der Schulter.

Die knöcherne Struktur des Schultergelenks, und nur auf diese soll hier eingegangen werden, besteht aus dem Kopf des Oberarmknochens (Humerus) E und der Gelenkpfanne (Glenoid) B des Schulterblatts A. Für die Funktion des Schultergelenks von Bedeutung sind außerdem zwei Knochenvorsprünge des Schulterblatts A, nämlich das Acromion C und das Coracoid D. Diese Knochenvorsprünge bilden zusammen mit einem sie verbindenden Band (nicht dargestellt) das so genannte Schulterdach, das eine gewölbeartige Form aufweist und ein Abwandern des Oberarmkopfes aus der Gelenkpfanne nach oben verhindert.

Fig. 1 zeigt eine so genannte anatomische Konfiguration, bei welcher die Prothese das Schultergelenk in seinem natürlichen Aufbau nachbildet, d.h. der Humerus E ist mit einem künstlichen Gelenkkopf 16 und das Schulterblatt A mit einer künstlichen Gelenkpfanne 14 versehen. Die Gelenkpfanne 14 ist hier nicht von einem erfindungsgemäßen künstlichen Gelenkteil gebildet, wobei dies aber möglich ist, wie eingangs bereits erläutert wurde. Fig. 1 soll hier nur einen Überblick geben.

Die Verankerung der Gelenkpfanne 14 am Schulterblatt A, genauer gesagt am entsprechend vorbereiteten Glenoid B, erfolgt im dargestellten Beispiel über Schrauben 14a. Die Verankerung des künstlichen Gelenkkopfes 16 am Humerus E erfolgt mittels eines Schaftes 12, der verschiedenartig ausgestaltet sein kann. Die konkrete Ausgestaltung des humerusseitigen Teils des künstlichen Schultergelenks ist nicht Gegenstand der Erfindung, so dass hierauf nachstehend nicht näher eingegangen wird.

Fig. 2 zeigt eine so genannte inverse Konfiguration, bei welcher der künstliche Gelenkkopf und die künstliche Gelenkpfanne 14' bezüglich ihrer Positionen im natürlichen Schultergelenk vertauscht sind. Der Gelenkkopf ist hier von einem erfindungsgemäßen künstlichen Gelenkteil gebildet, das eine erfindungsgemäße Basisplattform 11 und eine mit der Plattform 11 fest verbundene Kugelkomponente 17 umfasst. Die Verankerung der Plattform 11 am Schulterblatt A, genauer gesagt am entsprechend vorbereiteten Glenoid B, erfolgt im dargestellten Beispiel über eine konischen, mit Rippen versehenen Zapfen 19 der Plattform 11 sowie mittels polyaxialer Verankerungsschrauben 23, für die in der Plattform 11 entsprechende Schraubenaufnahmen vorgesehen sind. Der Zapfen 19 bildet eine Primärverankerung für die Plattform 11, wohingegen auf die eine - optionale - Sekundärverankerung bildenden Verankerungsschrauben 23 in Abhängigkeit von den jeweiligen Gegebenheiten auch verzichtet werden kann. Während die Plattform 11 hier also die künstliche Kugelkomponente 17 trägt, ist am Humerus E mittels eines Schaftes 12 eine künstliche Gelenkpfanne 14' verankert.

Besonderheiten der Erfindung sind u.a. die Ausgestaltung der Plattform 11 sowie die Art und Weise ihrer Anbringung am Glenoid B. Einzelheiten hierzu sowie zu dem konkreten Aufbau der Plattform 11 sowie zur jeweiligen künstlichen Gelenkkomponente 15 (Schale) bzw. 17 (Kugel) können der nachfolgenden Beschreibung entnommen werden.

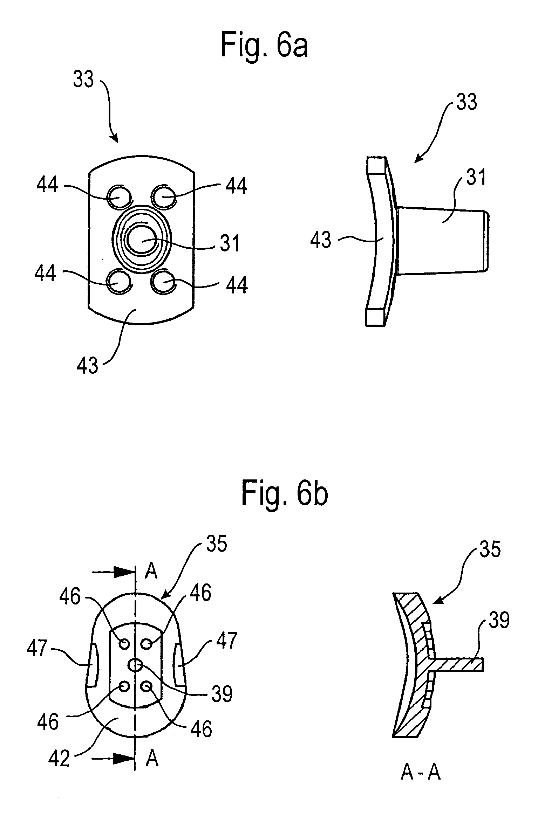

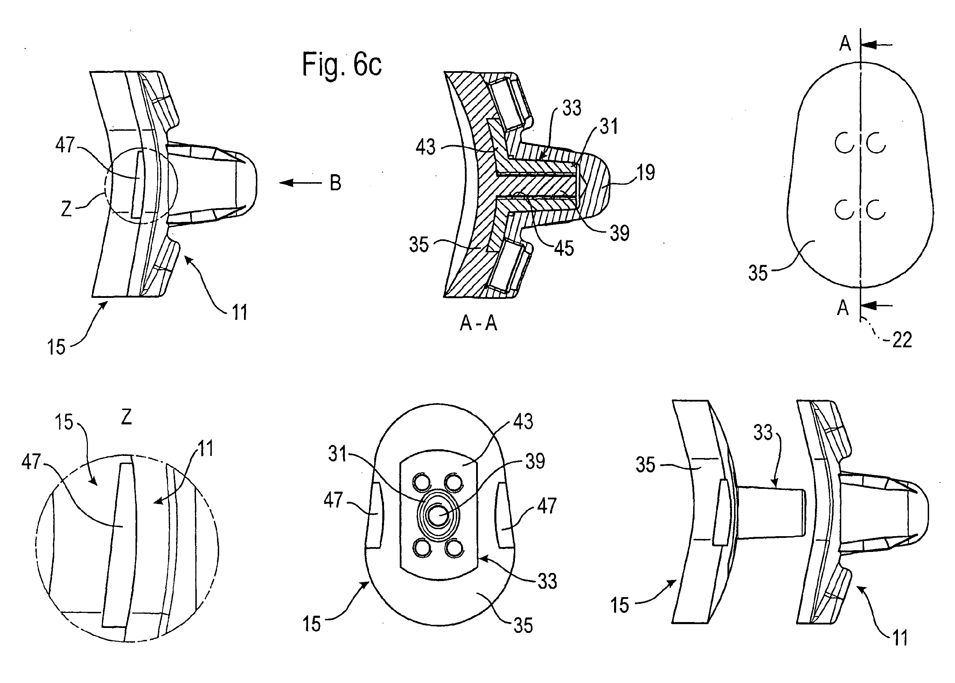

Die erfindungsgemäße Basisplattform 11 kann für alle nachstehend beschriebenen Varianten identisch sein. Die Plattform 11 ist jeweils einstückig und vollständig aus Metall hergestellt. Die jeweilige Gelenkkomponente 15, 17 (Fig. 3, 4 und 5) bzw. ein Schalenabschnitt 35 (Fig. 6, 7 und 8) oder Kugelabschnitt (nicht dargestellt), der zusammen mit einem metallischen Einsatz 33 die Gelenkkomponente 15, 17 bildet, können grundsätzlich jeweils entweder aus Kunststoff, beispielsweise aus Polyethylen, oder aus Metall hergestellt sein. Insbesondere die Kugelkomponente 17 bzw. der Kugelabschnitt sind aus Metall hergestellt.

Fig. 3 zeigt ein künstliches Gelenkteil für eine inverse Konfiguration. Eine Plattform 11 ist mit einer Kugelkomponente 17 verbunden. Die Plattform 11 umfasst ein schalenartiges Grundteil mit einer konvexen Seite und einer konkaven Seite. Von der konvexen Seite steht ein Zapfen 19 ab, der sich verjüngt und in diesem Ausführungsbeispiel eine konusförmige Au-ßenseite besitzt, die mit Längsrippen 20 versehen ist.

Des Weiteren stehen von der konvexen Seite zwei Kragen 25 ab, deren Mittelachsen jeweils mit einer Mittelachse eines in der Plattform 11 ausgebildeten Durchgangs 21 zusammenfallen. Die Durchgänge 21 sind als Schraubenaufnahmen 21 für polyaxiale Verankerungsschrauben 23 ausgebildet. Die Verankerungsschrauben 23 werden von der konkaven Seite der Plattform her eingeführt und mit Fixiermuttern 27, die mit einem Au-ßengewinde versehen sind, in den Schraubenaufnahmen 21 in einer jeweils gewünschten Orientierung relativ zur Plattform 11 fixiert. Hierzu sind die Schraubenaufnahmen 21 jeweils mit einem entsprechenden Innengewinde versehen. Die Fixiermuttern 27 klemmen die Köpfe 29 der Verankerungsschrauben 23 in den Schraubenaufnahmen 21 fest.

Die Mittelachsen der Schraubenaufnahmen 21 und damit der Verankerungskragen 25 weisen in unterschiedliche Richtungen, wobei diese Richtungen ausgehend von der konvexen Seite der Plattform 11 V-förmig auseinander laufen, und zwar symmetrisch zur Mittelachse des Zapfens 19. Wie die Darstellung unten rechts in Fig. 3 zeigt, liegen die Mittelachsen der Schraubenaufnahmen 21 und die Mittelachse des Zapfens 19 in einer gemeinsamen Ebene 22, die im Folgenden auch als Hauptebene bezeichnet wird.

Die Plattform 11 besitzt eine ovale Grundform mit entlang ihrer Längsachse abnehmender Breite, wobei eine Symmetrieebene, welche die Längsachse enthält, mit der vorstehend erwähnten Hauptebene 22 zusammenfällt.

Der konische Zapfen 19 besitzt eine elliptische äußere Querschnittsform, deren längere Hauptachse in der Hauptebene 22 liegt.

Die Kugelkomponente 17 ist mit einem Fixierabschnitt 31 versehen, der als konusförmiger Zapfen ausgebildet ist. Dabei weist der Fixierzapfen 31 der Kugelkomponente 17 ebenfalls eine elliptische äußere Querschnittsform auf. Zur Aufnahme des Fixierzapfens 31 ist der Zapfen 19 der Plattform 11 hohl ausgeführt und weist eine entsprechend dem Fixierzapfen 31 elliptisch ausgebildete innere Querschnittsform auf.

Zur Fixierung der Kugelkomponente 17 an der Plattform 11 wird die Kugelkomponente 17 mit ihrem Fixierzapfen 31 in die vom Zapfen 19 der Plattform 11 gebildete Konusaufnahme eingeschlagen, wodurch eine Presssitzverbindung hergestellt wird, bei der es sich insbesondere um einen Kegelpresssitz handelt.

Der Fixierzapfen 31 der Kugelkomponente 17 ist bezüglich eines Kugelabschnitts 37 exzentrisch angeordnet. Die Abweichung von der Kreisform bei dem Fixierzapfen 31 der Kugelkomponente 17 und dem Zapfen 19 der Plattform 11 ermöglicht eine gezielte Ausrichtung der Kugelkomponente 17 relativ zur Plattform 11. Eine mittige Anordnung des Fixierzapfens 31 ist grundsätzlich auch möglich.

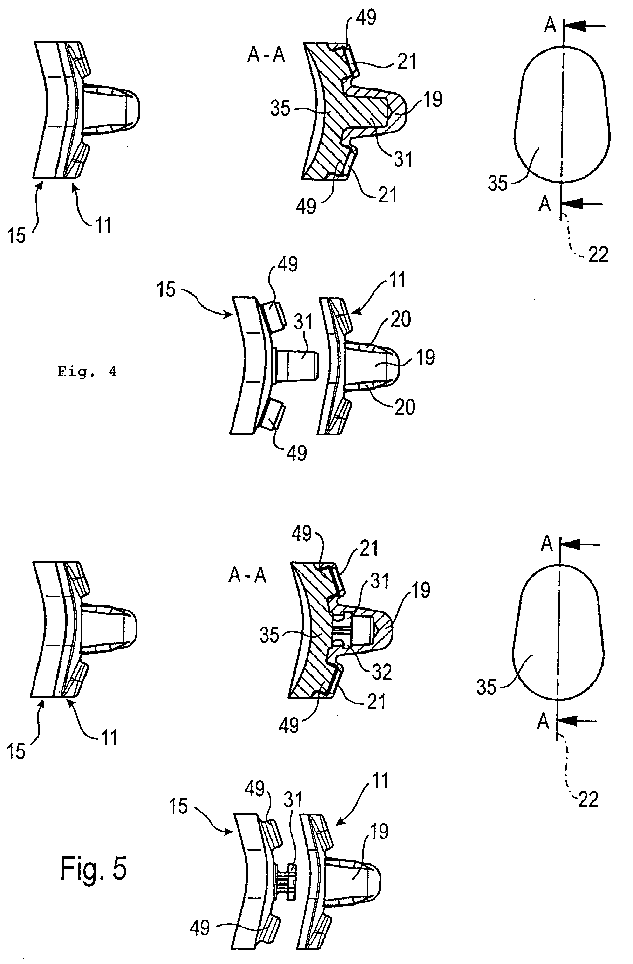

Die Fig. 4 und 5 zeigen Ausführungsbeispiele für eine anatomische Konfiguration. Die künstliche Gelenkkomponente ist als Schalenkomponente 15 ausgebildet, die über einen Fixierabschnitt 31 an der Plattform 11 fixiert ist.

Die beiden in den Fig. 4 und 5 dargestellten Varianten unterscheiden sich durch die Ausgestaltung ihres Fixierabschnitts 31, der in beiden Varianten einstückig mit dem Schalenabschnitt 35 ausgebildet ist.

Beim Ausführungsbeispiel der Fig. 4 ist der Fixierabschnitt 31 entsprechend der Ausführungsform von Fig. 3 als Konuszapfen ausgeführt, der in eine entsprechende Konusaufnahme des Zapfens 19 der Plattform 11 eingeschlagen wird, um eine reine kraftschlüssige Verbindung in Form eines Presssitzes herzustellen.

In der Variante gemäß Fig. 5 ist der Fixierabschnitt 31 gegenüber dem Ausführungsbeispiel der Fig. 4 verkürzt und an seinem freien Ende mit einem Ringvorsprung 32 versehen, der im zusammengesetzten Zustand in eine Ringnut eingreift, die auf der Innenseite des hohlen Zapfens 19 der Plattform 11 ausgebildet ist. Um eine elastische Verformbarkeit zu erreichen, kann der Fixierabschnitt 31 z.B. durch die Ausbildung von Schlitzen in eine Mehrzahl von auslenkbaren Segmenten unterteilt sein. Die Schalenkomponente 15 wird mit ihrem Fixierabschnitt 31 in den hohlen Zapfen 19 der Plattform 11 so weit eingesteckt, bis der Ringvorsprung 32 in die Ringnut einrastet.

Auch in den Ausführungsbeispielen der Fig. 4 und 5 können die innere Querschnittsform des Zapfens 19 und die äußere Querschnittsform des Fixierabschnitts 31 im Bereich des Ringvorsprungs 32 von einer Kreisform abweichen und beispielsweise elliptisch ausgebildet sein.

Die Ringnut im Zapfen 19 kann in einer konusförmigen Innenseite ausgebildet sein, die zur Aufnahme eines Konuszapfens 31 entsprechend den Ausführungsbeispielen der Fig. 3 und 4 geeignet ist, wodurch die Plattform 11 universell einsetzbar ist. Es hat sich herausgestellt, dass eine solche Ringnut die Herstellung einer Presssitzverbindung mit einem Konuszapfen nicht beeinträchtigt. Eine mit einer Ringnut im Zapfen 19 versehene Plattform 11 kann daher sowohl für eine kraftschlüssige Presssitzverbindung gemäß Fig. 3 und 4 als auch für eine formschlüssige Rast- oder Schnappverbindung gemäß Fig. 5 verwendet werden.

In beiden Varianten der Fig. 4 und 5 ist die künstliche Schalenkomponente 15 außerdem mit zwei Verankerungsvorsprüngen 49 versehen, die im zusammengesetzten Zustand in die Schraubenaufnahmen 21 der Plattform 11 eingreifen. Hierdurch wird eine besonders sichere Verbindung zwischen der künstlichen Gelenkkomponente 15 und der Plattform 11 erzielt, die bei der Herstellung des Gelenkteils durch Verpressen hergestellt werden kann.

Bei den Gelenkteilen gemäß Fig. 4 und 5 handelt es sich jeweils um eine werkseitige Ausgangskonfiguration zur Primärversorgung eines Patienten. Das Gelenkteil wird im zusammengesetzten Zustand implantiert, so dass eine Verankerung der Plattform 11 am Glenoid ausschließlich durch den Zapfen 19 erfolgt. Eine solche ausschließliche Primärverankerung ist in vielen Fällen vollkommen ausreichend.

Eine Konfiguration gemäß Fig. 4 und 5 ist grundsätzlich auch mit einer künstlichen Kugelkomponente anstelle der dargestellten Schalenkomponente 15 möglich.

Die aus Kunststoff bestehende Gelenkkomponente 15 kann von der Plattform 11 gelöst werden, und zwar ohne die Plattform 11 zu beschädigen. Das Lösen der Gelenkkomponente 15 kann bei bereits am Glenoid verankerter Plattform 11 erfolgen. Hierbei wird die Gelenkkomponente 15 durch Zerschneiden und/oder Aufbohren zerstört.

Die Schalenkomponente 15 kann anschließend durch eine andere Schalenkomponente z.B. gemäß den Ausführungsbeispielen der Fig. 6 - 8 ersetzt werden, wobei zuvor gegebenenfalls eine zusätzliche Sekundärverankerung der Plattform 11 am Glenoid durch polyaxiale Verankerungsschrauben erfolgen kann, die über die nunmehr zugänglichen Schrauben-' aufnahmen 21 eingeführt werden können. Es ist auch möglich, die Schalenkomponente 15 durch eine Kugelkomponente z.B. gemäß Fig. 3 zu ersetzen, wenn sich herausstellen sollte, dass die Schulter des Patienten für eine anatomische Prothesenkonfiguration, wie sie bei den Varianten der Fig. 4 und 5 gegeben ist, nicht geeignet ist.

Beim Ausführungsbeispiel der Fig. 6a - 6c ist die hier als Schale dargestellte künstliche Gelenkkomponente 15 mehrteilig ausgebildet. Die Schalenkomponente 15 umfasst einen aus Kunststoff hergestellten Schalenabschnitt 35 (Fig. 6b) und ein im Folgenden auch als Einsatz bezeichnetes separates Fixierstück 33 (Fig. 6a) aus Metall.

Das Fixierstück 33 besitzt einen plattenförmigen, gekrümmten Fußabschnitt 43, der mit einer Mehrzahl von Bohrungen 44 versehen ist. Die Bohrungen 44 können mit einem Innengewinde versehen sein. Der Fußabschnitt 43 und insbesondere die Bohrungen 44 dienen zur Befestigung des Einsatzes 33 am Schalenabschnitt 35. Diese Befestigung erfolgt durch Verpressen bei zuvor durch Erwärmen aufgeweichtem Kunststoffmaterial des Schalenabschnitts 35. Die Bohrungen 44 im Fußabschnitt 43 des Einsatzes 33 und - falls vorhanden - deren Innengewinde sorgen für eine innige formschlüssige Verbindung zwischen Einsatz 33 und Schalenabschnitt 35.

Des Weiteren ist der Fußabschnitt 43 mit einem konusförmigen Zapfen versehen, der einen Fixierabschnitt 31 des Einsatzes 33 bildet. Mit dem Fixierabschnitt 31 ist die Schalenkomponente 15 als Ganzes an der Plattform 11 verankerbar (Fig. 6c). In diesem Ausführungsbeispiel ist der Fixierabschnitt 31 des Einsatzes 33 als vollständiger Konus ausgebildet. Bei hergestellter Presssitzverbindung liegt folglich der konische Fixierabschnitt 31 vollflächig an der entsprechend konusförmigen Innenseite des hohlen Zapfens 19 der Plattform 11 an.

Wie der Schnitt A-A in Fig. 6c zeigt, ist der Einsatz 33 mit einem durchgehenden Innengewinde 45 versehen. Zum Lösen der Schalenkomponente 15 von der Plattform 11 kann nach zerstörendem Entfernen des Schalenabschnitts 35 ein beispielsweise als Abdrückschraube ausgebildetes Abdrückinstrument in den noch fest im Zapfen 19 der Plattform 11 sitzenden Einsatz 33 eingeschraubt werden, bis sich das Abdrückinstrument am Boden des Zapfens 19 abstützt und dann bei weitergehender Schraubbewegung den Einsatz 33 aus dem Zapfen 19 herausdrückt.

Das Innengewinde 45 des Einsatzes 33 kann abweichend von dem Ausführungsbeispiel der Fig. 6a - 6c vor dem freien Ende des Fixierabschnitts 31 enden.

Auch die Gewindebohrungen 44 des Fußabschnitts 43 können zum Abdrücken mittels eines einschraubbaren Abdrückinstrumentes genutzt werden.

Ein dornartiger Fortsatz 39 des Schalenabschnitts 35 dient als Träger für den Einsatz 33 und füllt das Innere des Fixierabschnitts 31 im Wesentlichen vollständig aus.

Des Weiteren ist der Schalenabschnitt 35 mit zwei taschenartigen Ablöseaussparungen 47 versehen. Die Aussparungen 47 sind jeweils seitlich nach außen und in Richtung der Plattform 11 offen und dienen zum Lösen des Schalenabschnitts 35 vom Einsatz 33. Hierzu können entsprechend geformte Instrumente in die Aussparungen 47 eingeführt werden, um unter Abstützung an der Plattform 11 Hebelkräfte auf den Schalenabschnitt 35 aufzubringen.

Die Lage und die Form der Ablöseaussparungen 47 sind insbesondere in der linken mittleren Darstellung der Fig. 6c zu erkennen, die eine Ansicht auf die Schalenkomponente 15 in Richtung B (Darstellung oben links in Fig. 6c) ohne die Plattform 11 ist.

Der Schalenabschnitt 35 (Fig. 6b) ist mit einer an den Fußabschnitt 43 des Einsatzes 33 (Fig. 6a) angepassten Vertiefung 42 versehen, die Vorsprünge 46 aufweist, welche mit den Bohrungen 44 des Fußabschnitts 43 ausgerichtet sind und beim Verpressen zur Befestigung des Einsatzes 33 am Schalenabschnitt 35 nach Art von Nieten verformt werden.

Die mehrteilige Ausführung der künstlichen Gelenkkomponente 15 mit dem aus Kunststoff bestehenden Gelenkabschnitt 35 und dem metallischen Einsatz 33 ermöglicht eine vorteilhafte Metall/Metall-Verbindung zwischen Gelenkkomponente 15 und Plattform 11.

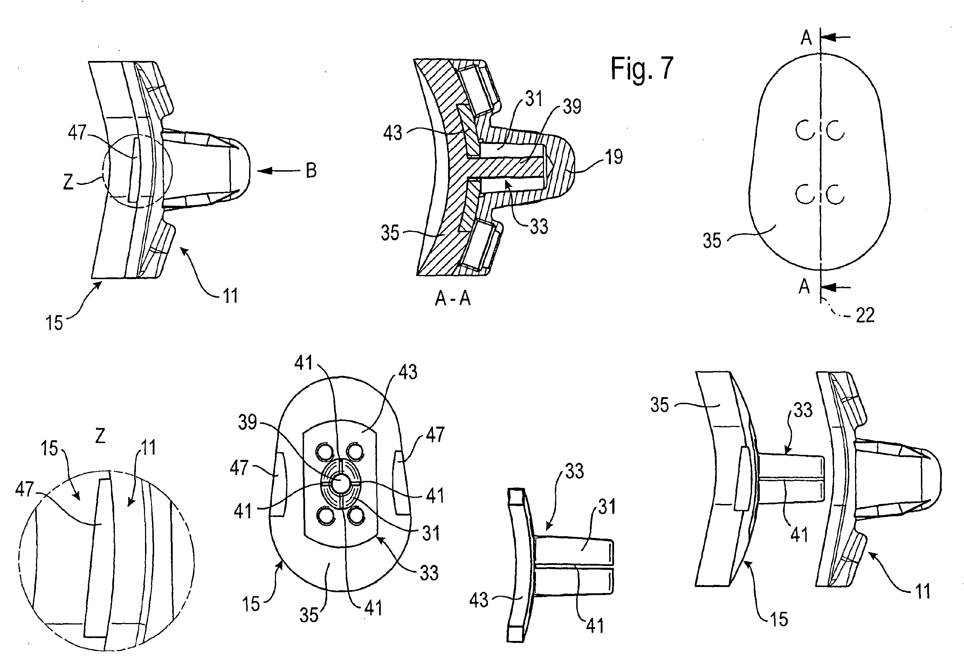

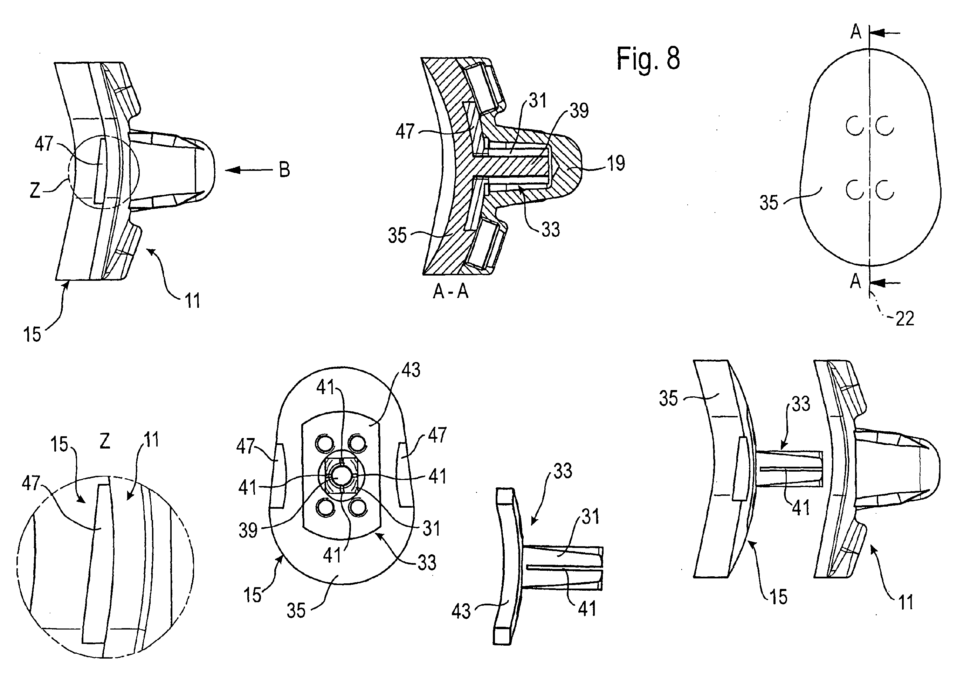

Die Ausführungsbeispiele der Fig. 7 und 8 unterscheiden sich von demjenigen der Fig. 6a - 6c jeweils lediglich durch die Ausgestaltung des konusförmigen Zapfens 31 des Einsatzes 33.

In der Variante der Fig. 7 ist der Fixierabschnitt 31 wiederum ein vollständiger Konus, der allerdings mit Längsschlitzen 41 versehen ist. Hierdurch entstehen unabhängig voneinander nachgiebige Segmente, wodurch sich die Anforderungen an die Herstellungstoleranzen für die konische Innenseite des Zapfens 19 der Plattform 11 verringern, ohne dass die Zuverlässigkeit der Presssitzverbindung zwischen dem Fixierabschnitt 31 und dem Zapfen 19 beeinträchtigt wird. Der Fortsatz 39 des Schalenabschnitts 35 kann ebenfalls mit Längsschlitzen versehen sein.

Ausgehend von dem Fußabschnitt 43 des Einsatzes 33 kann der Fixierabschnitt 31 über eine gewisse Länge zylindrisch ausgebildet sein, bevor er in eine Konusform übergeht und sich in Richtung seines freien Endes verjüngt. Hierdurch lässt sich erreichen, dass die einzelnen, durch die Längsschlitze 41 definierten Segmente des Fixierabschnitts 31 nicht im zylindrischen Bereich, sondern hauptsächlich im Bereich des freien Endes des Fixierabschnitts 31 ausgelenkt werden und insofern Biegefedern darstellen.

In dieser Variante kann vorgesehen sein, dass das Innengewinde des Einsatzes 33 für das Abdrückinstrument (vgl. Erläuterungen zum Ausführungsbeispiel der Fig. 6a - 6c) lediglich innerhalb des zylindrischen Bereichs des Fixierabschnitts 31 ausgebildet ist.

In der Variante der Fig. 8 ist der Fixierabschnitt 31 wiederum mit Längsschlitzen 41 versehen, wobei diese Variante auch ohne derartige Längsschlitze möglich ist.

Der Fixierabschnitt 31 ist hier nicht als vollständiger Konus ausgebildet, sondern besitzt eine äußere Querschnittsform, die in die innere Querschnittsform des Zapfens 19 der Plattform 11 einbeschrieben und hier rechteckig ausgebildet ist. Die Presssitzverbindung wird folglich nicht durch einen vollflächigen Kontakt zwischen dem Fixierabschnitt 31 und der Innenseite des Zapfens 19, sondern über mehrere linien- oder streifenförmige Kontaktstellen hergestellt, die diskret über den Umfang verteilt sind. Aufgrund der geringeren Anforderungen an die Fertigungstoleranzen ist diese Variante noch einfacher herstellbar.

### Bezugszeichenliste

- 11: Plattform für Glenoid
- 12: Schaft
- 14: künstliche Gelenkpfanne für Glenoid
- 14': künstliche Gelenkpfanne für Humerus
- 14a: Schraube
- 15: Schalenkomponente für Glenoid
- 16: künstlicher Gelenkkopf für Humerus
- 17: Kugelkomponente für Glenoid
- 19: Zapfen
- 20: Rippe
- 21: Schraubenaufnahme
- 22: Hauptebene
- 23: polyaxiale Verankerungsschraube
- 25: Verankerungskragen
- 27: Fixiermutter
- 29: Kopf der Verankerungsschraube
- 31: Fixierabschnitt
- 32: Ringvorsprung
- 33: Fixierstück, Einsatz
- 35: Schalenabschnitt
- 37: Kugelabschnitt
- 39: Fortsatz
- 41: Längsschlitz
- 42: Vertiefung
- 43: Fußabschnitt
- 44: Bohrung
- 45: Innengewinde
- 46: Vorsprung
- 47: Ablöseaussparung
- 49: Verankerungsvorsprung

- A: Schulterblatt
- B: Gelenkpfanne (Glenoid)
- C: Acromion
- D: Coracoid
- E: Oberarmknochen (Humerus)

## Patentansprüche

1. Basisplattform für ein künstliches Gelenk, insbesondere für ein künstliches Schultergelenk,
wobei die Plattform (11) am Knochen (B) verankerbar ist und als Träger für eine künstliche Gelenkkomponente (15, 17) dient, die sowohl in Form einer Schalenkomponente (15) für eine anatomische Konfiguration als auch in Form einer Kugelkomponente (17) für eine inverse Konfiguration vorgesehen sein kann, und
wobei an der Plattform (11) verschiedene Gelenkkomponenten (15, 17) montierbar sind.

2. Basisplattform nach Anspruch 1,
**dadurch gekennzeichnet,**
**dass** die Plattform (11), insbesondere für eine Primärverankerung im Knochen (B), einen Zapfen (19) aufweist.

3. Basisplattform nach Anspruch 1 oder 2,
**dadurch gekennzeichnet,**
**dass** der Zapfen (19) eine sich, insbesondere konusförmig, verjüngenden Außenseite aufweist.

4. Basisplattform nach einem der vorhergehenden Ansprüche,
**dadurch gekennzeichnet,**
**dass** der Zapfen (19) zumindest über einen Teil seiner Länge eine von einer Kreisform abweichende, insbesondere ovale oder elliptische, äußere Querschnittsform aufweist.

5. Basisplattform nach einem der vorhergehenden Ansprüche,
**dadurch gekennzeichnet,**
**dass** die Plattform (11) für eine Sekundärverankerung im Knochen (B) Schraubenaufnahmen (21) für polyaxiale Verankerungsschrauben (23) aufweist.

6. Basisplattform nach Anspruch 5,
**dadurch gekennzeichnet,**
**dass** eine Mittelachse des Zapfens (19) und Mittelachsen wenigstens zweier Schraubenaufnahmen (21) in einer gemeinsamen Hauptebene (22) liegen.

7. Basisplattform nach Anspruch 6,
**dadurch gekennzeichnet,**
**dass** der Zapfen (19) zumindest über einen Teil seiner Länge eine ovale oder elliptische äußere Querschnittsform aufweist, deren eine, insbesondere lange, Hauptachse in der gemeinsamen Hauptebene (22) liegt.

8. Basisplattform nach einem der Ansprüche 5 bis 7,
**dadurch gekennzeichnet,**
**dass** Mittelachsen wenigstens zweier Schraubenaufnahmen (21) in unterschiedliche Richtungen weisen.

9. Basisplattform nach einem der Ansprüche 5 bis 8,
**dadurch gekennzeichnet,**
**dass** die Schraubenaufnahmen (21) jeweils einen Verankerungskragen (25) aufweisen, der auf der gleichen Seite wie ein Zapfen (19) von der Plattform (11) absteht.

10. Basisplattform nach einem der Ansprüche 2 bis 9,
**dadurch gekennzeichnet,**
**dass** der Zapfen (19) an seiner Außenseite mit im Wesentlichen in Längsrichtung verlaufenden Rippen (20) versehen ist.

11. Basisplattform nach einem der vorhergehenden Ansprüche,
**dadurch gekennzeichnet,**
**dass** die Plattform (11) aus Metall hergestellt ist.

12. Basisplattform nach einem der vorhergehenden Ansprüche,
**dadurch gekennzeichnet,**
**dass** die Plattform (11) im Randbereich ihrer bei verankerter künstlicher Gelenkkomponente (15, 17) der Gelenkkomponente (15, 17) zugewandten Seite wenigstens eine von außen zugängliche, insbesondere taschenartige Ablöseaussparung aufweist, in die oder an der ein Ablöseinstrument zum Lösen der Verankerung der Gelenkkomponente (15, 17) an der Plattform (11) einbringbar bzw. ansetzbar ist.

13. Basisplattform nach einem der Ansprüche 2 bis 12,
**dadurch gekennzeichnet,**
**dass** der Zapfen (19) hohl ausgeführt und zur Aufnahme eines Fixierabschnitts (31) der künstlichen Gelenkkomponente (15, 17) ausgebildet ist, mit dem die künstliche Gelenkkomponente (15, 17) an der Plattform (11) verankerbar ist.

14. Basisplattform nach Anspruch 13,
**dadurch gekennzeichnet,**
**dass** die innere Querschnittsformgebung des Zapfens (19) der äußeren Querschnittsformgebung des Zapfens (19) entspricht.

15. Basisplattform nach Anspruch 13 oder 14,
**dadurch gekennzeichnet,**
**dass** der Zapfen (19) zumindest über einen Teil seiner Länge eine von einer Kreisform abweichende, insbesondere ovale oder elliptische, innere Querschnittsform aufweist.

16. Basisplattform nach einem der Ansprüche 13 bis 15,
**dadurch gekennzeichnet,**
**dass** der Zapfen (19) zur Herstellung einer, vorzugsweise selbst hemmenden, Klemm- oder Presssitzverbindung, insbesondere eines Kegelpresssitzes, oder einer Rast- oder Schnappverbindung mit dem Fixierabschnitt (31) der künstlichen Gelenkkomponente (15, 17) ausgebildet ist.

17. Gelenkkomponente für ein künstliches Gelenk, insbesondere für ein künstliches Schultergelenk,
wobei die Gelenkkomponente (15, 17) entweder als Schalenkomponente (15) für eine anatomische Konfiguration oder als Kugelkomponente (17) für eine inverse Konfiguration ausgebildet ist und einen Fixierabschnitt (31) aufweist, mit dem die Gelenkkomponente (15, 17) an einem Zapfen (19), insbesondere im Inneren eines hohlen Zapfens (19), einer Basisplattform (11) nach einem der Ansprüche 1 bis 16 verankerbar ist.

18. Gelenkkomponente nach Anspruch 17,
**dadurch gekennzeichnet,**
**dass** der Fixierabschnitt (31) zur Herstellung einer, vorzugsweise selbst hemmenden, Klemm- oder Presssitzverbindung, insbesondere eines Kegelpresssitzes, oder einer Rast- oder Schnappverbindung mit dem Zapfen (19) der Plattform (11) ausgebildet ist, wobei vorzugsweise der Fixierabschnitt (31) eine sich, insbesondere konusförmig, verjüngende Außenseite aufweist.

19. Gelenkkomponente nach Anspruch 17 oder 18,
**dadurch gekennzeichnet,**
**dass** der Fixierabschnitt (31) zur Herstellung eines Kontaktes mit der Innenseite des Zapfens (19) der Plattform (11) ausgebildet ist,
wobei der Kontakt im Wesentlichen über den gesamten Umfang erfolgt.

20. Gelenkkomponente nach Anspruch 17 oder 18,
**dadurch gekennzeichnet,**
**dass** der Fixierabschnitt (31) zur Herstellung eines Kontaktes mit der Innenseite des Zapfens (19) der Plattform (11) ausgebildet ist,
wobei der Kontakt zumindest über einen Teil der Länge des Fixierabschnitts (31) und/oder der Innenseite des Zapfens (19) an mehreren diskret über den Umfang verteilten, linien- oder streifenförmigen Bereichen erfolgt.

21. Gelenkkomponente nach einem der Ansprüche 17 bis 20,
**dadurch gekennzeichnet,**
**dass** der Fixierabschnitt (31) von einem separaten Fixierstück (33) gebildet ist, das mit einem Schalen- oder Kugelabschnitt (35, 37) der Gelenkkomponente (15, 17) fest verbunden ist, wobei über das Fixierstück (33) eine Verbindung zwischen dem Schalen- bzw. Kugelabschnitt (35, 37) und dem Zapfen (19) der Plattform (11) herstellbar ist.

22. Gelenkkomponente nach Anspruch 21,
**dadurch gekennzeichnet,**
**dass** das separate Fixierstück (33) aus Metall hergestellt ist.

23. Gelenkkomponente nach Anspruch 21 oder 22,
**dadurch gekennzeichnet,**
**dass** das separate Fixierstück (33) kappen- oder hülsenförmig ausgebildet ist und einen Fortsatz (39) des Schalen- bzw. Kugelabschnitts (35, 37) umgibt.

24. Gelenkkomponente nach Anspruch 23,
**dadurch gekennzeichnet,**
**dass** der Fortsatz (39) des Schalen- oder Kugelabschnitts (35, 37) als ein das Innere des separaten Fixierstücks (33) zumindest im Wesentlichen ausfüllender Trägerkern ausgebildet ist, wobei vorzugsweise der Trägerkern mit einem oder mehreren Längsschlitzen (41) versehen ist.

25. Gelenkkomponente nach einem der Ansprüche 21 bis 24,
**dadurch gekennzeichnet,**
**dass** das separate Fixierstück (33) in Bezug auf Kräfte, die bei der Herstellung einer Klemm- oder Presssitzverbindung mit dem Zapfen (19) der Plattform (11) wirksam sind, nachgiebig ausgebildet ist.

26. Gelenkkomponente nach einem der Ansprüche 21 bis 25,
**dadurch gekennzeichnet,**
**dass** das separate Fixierstück (33) mit einem oder mehreren Längsschlitzen (41) versehen ist.

27. Gelenkkomponente nach einem der Ansprüche 21 bis 26,
**dadurch gekennzeichnet,**
**dass** das separate Fixierstück (33) einen flanschartigen Fußabschnitt (43) aufweist, über den das Fixierstück (33) mit dem Schalen- oder Kugelabschnitt (35, 37) verbunden ist.

28. Gelenkkomponente nach einem der Ansprüche 21 bis 27,
**dadurch gekennzeichnet,**
**dass** die Verbindung zwischen dem separaten Fixierstück (33) und dem Schalen- oder Kugelabschnitt (35, 37) eine durch Verpressen hergestellte formschlüssige Verbindung ist.

29. Gelenkkomponente nach einem der Ansprüche 17 bis 28,
**dadurch gekennzeichnet,**
**dass** die Gelenkkomponente (15, 17) derart ausgebildet ist, dass die Verankerung der Gelenkkomponente (15, 17) an der Plattform (11) ohne Beschädigung der Plattform (11) lösbar ist.

30. Gelenkkomponente nach einem der Ansprüche 17 bis 29,
**dadurch gekennzeichnet,**
**dass** die Gelenkkomponente (15, 17) mit wenigstens einem Gewindeabschnitt (44, 45) für ein einschraubbares Abdrückinstrument versehen ist, wobei vorzugsweise der Gewindeabschnitt (44) in Form eines Innengewindes (45) des Fixierabschnitts (31) und/oder einer Gewindebohrung (44) in einem flanschartigen Fußabschnitt (43) eines separaten Fixierstücks (33) vorgesehen ist.

31. Gelenkkomponente nach einem der Ansprüche 17 bis 30,
**dadurch gekennzeichnet,**
**dass** die Gelenkkomponente (15, 17) im Randbereich ihrer im verankerten Zustand der Plattform (11) zugewandten Seite wenigstens eine von außen zugängliche, insbesondere taschenartige Ablöseaussparung (47) aufweist, in die oder an der ein Ablöseinstrument zum Lösen der Verankerung der Gelenkkomponente (15, 17) an der Plattform (11) einbringbar bzw. ansetzbar ist.

32. Gelenkkomponente nach einem der Ansprüche 17 bis 31,
**dadurch gekennzeichnet,**
**dass** die Gelenkkomponente (15, 17) mit Verankerungsvorsprüngen (49) versehen ist, die in Schraubenaufnahmen (21) der Plattform (11) eingreifen.

33. Gelenkteil für ein künstliches Gelenk, insbesondere für ein künstliches Schultergelenk,
mit einer Basisplattform (11) nach einem der Ansprüche 1 bis 16
und mit einer künstlichen Gelenkkomponente (15, 17) nach einem der Ansprüche 17 bis 32.

34. Bausatz zur Bildung unterschiedlicher Gelenkteile für ein künstliches Gelenk, insbesondere für ein künstliches Schultergelenk,
mit zumindest einer Plattform (11) nach einem der Ansprüche 1 bis 16, und
mit mehreren unterschiedlichen künstlichen Gelenkkomponenten (15, 17) nach einem der Ansprüche 17 bis 32, die jeweils zur Bildung eines Gelenkteils entweder für eine anatomische Konfiguration oder für eine inverse Konfiguration an der Plattform (11) verankerbar sind.

35. Bausatz nach Anspruch 34,
**dadurch gekennzeichnet,**
**dass** eine an der Plattform (11) verankerte künstliche Gelenkkomponente (15, 17) gegen eine andere künstliche Gelenkkomponente (15, 17) austauschbar ist, wobei ein Wechsel von einer anatomischen Konfiguration zu einer inversen Konfiguration möglich ist, und umgekehrt.

36. Verfahren zum Implantieren eines künstlichen Gelenks, insbesondere eines künstlichen Schultergelenks, bei dem ein Gelenkteil (11, 15, 17) aus einer Basisplattform (11) nach einem der Ansprüche 1 bis 16 und einer künstlichen Gelenkkomponente (15, 17) nach einem der Ansprüche 17 bis 32 in einer Ausgangskonfiguration vorliegt und diese Ausgangskonfiguration in eine Endkonfiguration geändert wird, indem eine an der Plattform (11) des Gelenkteils verankerte künstliche Gelenkkomponente (15, 17) gegen eine andere künstliche Gelenkkomponente (15, 17) ausgetauscht wird.

37. Verfahren nach Anspruch 36,
**dadurch gekennzeichnet,**
**dass** die Ausgangskonfiguration eine anatomische Konfiguration und die Endkonfiguration eine inverse Konfiguration ist, oder umgekehrt.

38. Verfahren nach Anspruch 36 oder 37,
**dadurch gekennzeichnet,**
**dass** die Konfigurationsänderung ohne Beschädigung der Plattform (11) erfolgt.

39. Verfahren nach einem der Ansprüche 36 bis 38,
**dadurch gekennzeichnet,**
**dass** die Ausgangskonfiguration dem Liefer- oder Werkszustand des Gelenkteils (11, 15, 17) vor der Erstimplantation entspricht.

40. Verfahren nach einem der Ansprüche 36 bis 39,
**dadurch gekennzeichnet,**
**dass** das Gelenkteil (11, 15, 17) in der Ausgangskonfiguration bereits implantiert ist.
